(19) Europäisches Patentamt
European Patent Office
Office européen des brevets

(11) Numéro de publication: **0 199 644 B1**

(12) **FASCICULE DE BREVET EUROPEEN**

(45) Date de publication du fascicule du brevet: **08.02.89**

(51) Int. Cl.⁴: **C 12 N 11/06**

(21) Numéro de dépôt: **86400825.5**

(22) Date de dépôt: **17.04.86**

(54) **Perfectionnement à l'immobilisation d'enzymes.**

(30) Priorité: **19.04.85 FR 8505939**

(43) Date de publication de la demande: **29.10.86 Bulletin 86/44**

(45) Mention de la délivrance du brevet: **08.02.89 Bulletin 89/6**

(84) Etats contractants désignés: **CH DE GB IT LI NL SE**

(56) Documents cités:
**CHEMICAL ABSTRACTS, vol. 90, no. 15, 9 avril 1979, page 676, no. 121890g, Columbus, Ohio, US; V.C. BORLAZA et al.: "Evaluation of dithiobis(thioformate) and S-methyl dithiocarbonate as reactive groups for the immobilization of biological molecules", & CARBOHYDR. RES. 1979, 68(1), 141-3**
**CARBOHYDRATE RESEARCH, vol. 79, 1980, pages 125-132, Elsevier Scientific Publishing Co., Amsterdam, NL; V.C. BORLAZA et al.: "Enzyme immobilization on cellulose dithiobis(thioformate) and cellulose S-methyldithiocarbonate"**

(73) Titulaire: **SOCIETE NATIONALE ELF AQUITAINE Société anonyme dite, Tour Elf 2, Place de la Coupole La Défense 6, F-92400 Courbevoie (FR)**

(72) Inventeur: **Levesque, Guy, 19 rue de l'Unité, Epron F-14610 Thanon (FR)**
Inventeur: **Seris, Jean-Louis, Lot. Parenche No. 15 Chemin Vignats, F-64110 Jurancon (FR)**

(74) Mandataire: **Kohn, Armand, 5 Avenue Foch, F-92380 Garches (FR)**



Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

**Description**

La présente invention concerne un nouveau mode perfectionné d'immobilisation d'enzymes sur supports solides. Elle comprend le procédé d'immobilisation, ainsi que les enzymes immobilisées obtenues.

L'immobilisation des enzymes, c'est-à-dire leur fixation sur un support solide, éventuellement plastique, a constitué un progrès important dans cette branche de la technique. Elle a apporté une amélioration considérable, en ce qu'elle a permis de réutiliser de nombreuses fois une enzyme qui, autrement, à l'état libre, était pratiquement perdue d'une opération à l'autre. En outre, le travail avec une enzyme ainsi immobilisée contriobue à la pureté du milieu traité. Cependant, la fixation des enzymes sur des supports solides, que ce soit par adsorption ou par liaisons covalents, est une opération assez délicate, nécessitant une choix bien étudié des matières. L'immobilisation par liaisons covalentes conduit à des systèmes plus stables, évitant des pertes d'enzymes pendant leur utilisation. Mais le système enzymatique, obtenu par cette dernière méthode, présente généralement une activité plus faible que l'enzyme libre, seule, à cause de l'encombrement stérique; en effet, la fixation d'une macromolécule protéique sur un support rigide réduit l'accessibilité du substrat à traiter aux actifs de la macromolécule. On sait par exemple que la ribonucléase immobilisée sur agarose subit une baisse d'activité de l'ordre de 10 à 75% par rapport à l'enzyme libre, selon la nature du substrat traité. Pour remédier à ce défaut on peut utiliser un «bras de support», c'est-à-dire un réactif intermédiaire, combiné d'une part au support et de l'autre à l'enzyme. Toutefois, même avec les systèmes connus de ce genre, l'activité enzymatique est généralement inférieure à celle de l'enzyme libre. D'autre part, le choix d'agents pouvant jouer le rôle de «bras» est assez restreint, car beaucoup de réactifs éventuels altèrent l'activité de l'enzyme, ou bien les conditions opératoires qu'ils impliquent ne sont pas compatibles avec la conservation de l'activité catalytique. Les agents les plus couramment utilisés à l'heure actuelle sont tels que le glutaraldéhyde, bromure de cyanogène et quelques autres rappelés ci-dessous.

| Groupe fonctionnel | | Méthode d'activation |
|---|---|---|
| enzyme | support | |
| $-NH_2$ | $-NH_2$ | glutaraldéhyde |
| $-NH_2$ | $-COOH$ | azoture d'acyle carbodiimide |
| $-NH_2$ | $-OH$ | bromure de cyanogène triazines |
| $-COOH$ | $-NH_2$ | carbodiimide |
| 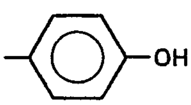 | 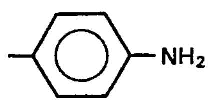 | sel de diazonium |
| $-SH$ | $-SH$ | pont disulfure |

Or la plupart de ces agents sont d'une application délicate et comportent les inconvénients mentionnés plus haut.

La présente invention apporte à la technique de l'immobilisation des enzymes on progrès marqué: elle utilise un nouvel agent, servant de «bras de support», dont l'application est plus aisée et plus sûre que celle des agents connus, indiqués plus haut; d'autre part, elle apporte l'important avantage de procurer un rendement élevé en activité de l'enzyme, après l'immmobilisation de celle-ci. Les systèmes enzymatiques, formés suivant linvention, sont très stables et faciles à stocker à des températures normales.

Le procédé d'immobilisation d'enzyme par liaisons covalentes, suivant la présente invention, qui utilise un support comportant des groupes $NH_2$ et un agent intermédiaire se fixant, à la fois par ces groupes $NH_2$ sur le support, ainsi que sur l'enzyme, est caractérisé en ce que l'agent intermédiaire, ou «bras de support», est constitué par un ester bis-dithioïque, dont les deux fonctions ester se terminent chacune par un groupe nucléofuge.

Autrement dit, les agents d'activation du support et de l'enzyme sont des bis-dithioesters porteurs de 2 atomes ou groupes nucléofuges susceptibles de réagir avec des groupes $NH_2$ du support et de l'enzyme.

L'invention résulte de la constatation surprenante que la présence des groupes dithioïque facilite grandement la fixation de ces bis-dithioesters tant sur des supports que sur des enzymes, sans nécessiter une activation spéciale comme c'est le cas des agents de la technique connue, en particulier de diacides ou leurs anhydrides.

Le réactif, suivant l'invention, peut être représenté par la formule:

X-R'-S-C-R-C-S-R'-X (1)
      ||    ||
      S    S

où R est une chaîne ou cycle hydrocarboné, à nombre d'atomes de C variable, mais de préférence 1 à 20; il en est de même des groupes R'; quant à X il désigne un atome ou groupe pouvant favoriser la réaction avec -NH ou $-NH_2$, tel que par exemple un halogène, carboxyle, sulfinique, sulfonique, phosphoreux, phosphoneux, phosphorique, phosphonique, H actif, SH, amide, hydroxy, etc.; lorsque R est une chaîne aliphatique, celle-ci peut être saturée ou non saturée et comprend le plus souvent 2 à 18 atomes de carbone et plus particulièrement 6 à 12. Cette chaîne peut porter des substituants, notamment des groupes aryliques, alkyliques éthers ou/et halogénes. Comme R peut être un groupe cyclique, il peut être constitué par des cycloalcanes, cycloalcènes, ou/et aryles à 1 ou 2 noyaux, mais surtout en $C_5$ à $C_{12}$, en particulier phényle, alkylphényles, diphényle, ou de tels cycles porteurs des substituants.

Un groupe X de choix, pour la réalisation de l'invention, est le groupement carboxylique COOH qui permet de faire réagir le «bras» avec le support et l'enzyme dans des conditions suffisamment douces et pourtant efficacement.

Voici, à titre d'exemples non limitatifs, quelques uns des bis (dithio-esters de carboxy-alkyles) ou tétrathioalcane dithioates de carboxyalkyles.

I $HOOC\text{-}CH_2\text{-}S\text{-}C\text{-}CH_2\text{-}C\text{-}S\text{-}CH_2COOH$
      ||    ||
      S    S

II $HOOC\text{-}CH_2S\text{-}C\text{-}(CH_2)_6\text{-}C\text{-}S\text{-}CH_2COOH$
      ||    ||
      S    S

III $HOOC\text{-}CH_2CH_2CH_2\text{-}S\text{-}C\text{-}(CH_2)_3\text{-}C\text{-}S\text{-}CH_2CH_2CH_2COOH$
      ||    ||
      S    S

IV $HOOC\text{-}CH_2CH_2\text{-}S\text{-}C\text{-}(CH_2)_{10}\text{-}C\text{-}S\text{-}CH_2CH_2COOH$
      ||    ||
      S    S

V $HOOC\text{-}(CH_2)_6\text{-}S\text{-}C\text{-}CH_2CH_2\text{-}C\text{-}S\text{-}(CH_2)_6COOH$
      ||    ||
      S    S

VI $HOOC—CH_2\text{-}S\text{-}C\text{-}(CH_2)_{12}\text{-}C\text{-}S\text{-}CH_2\text{-}COOH$
      ||    ||
      S    S

VII 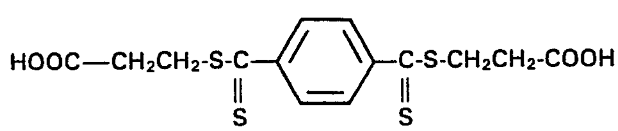

VIII 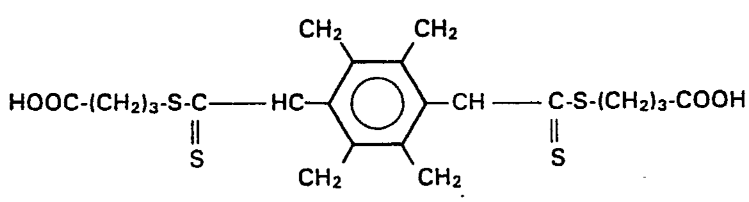

IX 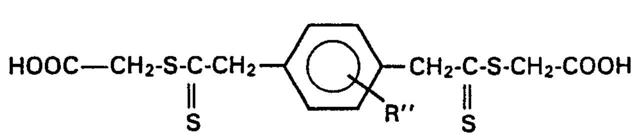

X $HOOC-CH_2-S-C-CH_2CH_2-O-CH_2CH_2-C-CH_2-COOH$
|| ||
S S

XI $HOOC-CH_2CH-CH_2-SC-(CH_2)_8-CS-CH_2CHCH_2-COOH$
| || || |
Cl S S Cl

Dans le composé IX R'' peut être absent ou bien formé par un substituant tel qu'alogène, nitro, alkyl, alkoxy, etc. Il peut d'ailleurs y avoir plusieurs de tels substituants R''.

Parmi les autres esters dithioïques, pouvant être utilisés pour l'immobilisation d'enzymes, on peut citer les formules typiques suivantes:

XII $H_2NOC-CH_2-S-C-(CH_2)_8-C-S-CH_2-CONH_2$
|| ||
S S

XIII $Cl_3CCH_2-S-C-(CH_2)_4-C-SCH_2CCl_3$
|| ||
S S

XIV $HO_2S-(CH_2)_4-S-C-(Ph)-C-S-(CH_2)_4-SO_2H$
|| ||
S S

XV $HO_3S-CH_2-S-C-(CH_2)_{10}-C-S-CH_2-SO_3H$
|| ||
S S

XVI $HOCH_2CH_2-S-C-(CH_2)_6-C-S-CH_2CH_2OH$
|| ||
S S

XVII

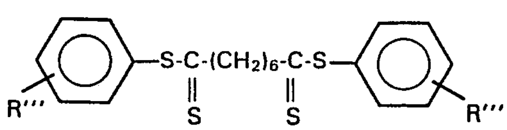

XVIII

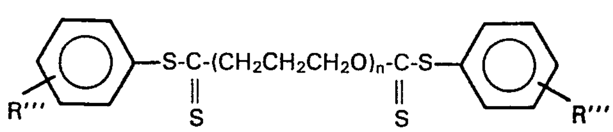

XIX $(CH_3)_2N-CH_2CH_2-S-C(CH_2)_3-C-S-CH_2CH_2-N(CH_3)_2$
|| ||
S S

XX $HS(CH_2)_3-S-C-(CH_2)_3-C-S-(CH_2)_3-SH$
|| ||
S S

Ces différents composés peuvent être préparés à l'aide des méthodes comme celles de LEVESSON (Act. Chem. Scand. part B, 29. 539, 1975), de MARVEL et col. (J. Am. Chem. Soc. 77, 5997-1955) ou/et de GRESSIER et LEVESQUE (Europ. Polymer. 16, 1093-1980).

D'après ce qui précède, on voit que l'invention permet de choisir, pour l'agent de fixation, un dithioester formant une chaîne plus ou moins longue: on peut ainsi se servir d'un «bras» aussi long qu'il le faut pour réduire ou supprimer l'empêchement stérique lors de l'action de l'enzyme immobilisée sur un substrat donné. En outre, les propriétés de l'agent de fixation peuvent être modifiées selon les applications envisagées; cela peut se faire par le choix approprié des groupes R, R', R'', R''' et X. Ainsi, par exemple, un noyau aromatique en R procure de la rigidité au bras support; une chaîne aliphatique apporte la souplesse; des alkoxy, tels que $-(CH_2CH_2O)_n$ et similaires, conduisent à une hydrophilie plus au moins prononcé, tandis que des longues chaînes de $CH_2$ ou d'aryles, tels que diphényle, tendent à l'hydrophobie.

On peut aussi moduler l'activité nucléofuge de l'ester dithioïque rendant celui-ci compatible avec la fragilité de l'enzyme donnée.

Le nouvel agent intermédiaire, suivant l'invention, peut être combiné d'abord avec le support ou bien avec l'enzyme, sans que celui-ci subisse une dénaturation.

Les supports, pouvant être employés, sont en principe tous ceux qui sont susceptibles de fixer, d'une manière ou d'une autre, des groupes $NH_2$, ou bien ceux dont la composition comprend déjà des $NH_2$ disponibles pour la réaction avec l'agent dithioïque. Ainsi peut-on employer des matières comme silice, divers silicates, entre autres zéolithes, verre, alumino-silicate, bentonite, ainsi que des alumines et charbons actifs traités de manière appropriée par une amine. De même conviennent des celluloses et leurs dérivés, notamment celluloses aminées, chitosane et surtout des protéines suffisamment rigides porteuses de groupes NH ou $NH_2$, susceptibles de réagir avec les groupes X de l'agent suivant l'invention. Dans cette dernière catégorie, il est possible de choisir le support adéquat parmi les matières telles que par exemple caséine, lactoglobuline, ovalbumine, sérum albumine, etc., éventuellement durcies par chauffage ou chimiquement. Une autre classe de supports fort intéressants sont des polymères synthétiques portant des $NH_2$ libres ou partiellement combinés, comme en particulier polyaminopolystyrène, polyacrylamide, polyacrylates aminés, polyméthacrylate (ou polyacrylate) de glycidyle aminé par ouverture des ponts époxy, sous l'effet d'une amine ou de l'ammoniac (FR 2 442 244).

En ce qui concerne les enzymes et coenzymes justiciables du procédé de l'invention, elles appartiennent d'une façon générale à tous les groupes, c'est-à-dire aux hydrolases, notamment protéases, carboxydrases et estérases, de même qu'aux oxydases, deshydrogénases, aux enzymes d'isomérisation et celles de polymérisation, etc. On peut ainsi immobiliser avec l'aide de dithioesters suivant l'invention, les amylases, l'arginase, la dipetidase, l'énolase, l'aldolase, l'adénosine, désaminase, les lipases, les catalases, peroxydases, la glucose oxydase, galactose oxydase, lactates oxydases, oxylate oxydase, pyruvate oxydase, nucléase, ribonucléase, lysozyme, trypsine, chymotrypsine, présure, invertase, maltase, pectinase, alcool déshydrogénases, lactate déhydrogénase, formiate déshydrogénase, glucose déshydrogénase etc.

L'immobilisation, suivant l'invention, des enzymes peut être réalisée par simple contact, à une température fraîche, le plus souvent entre 0° et 30°C, et surtout entre 0° et 10°C, de l'enzyme voulue avec le support activé par le dithioester, au sein d'un tampon de pH convenant à l'nzyme.

L'activation préalable du support s'opère généralement par le maintien de ce support dans une solution de dithioester dans un solvant de celui-ci. Selon l'affinité du dithioester pour les groupes $-NH_2$ du support, cette opération a lieu à froid ou à chaud, généralement entre 0° et 100°C, le plus souvent à la température ambiante ou entre 10° et 35°C.,

Lorsque le support ne comprend pas naturellement des $-NH_2$ ou des -NH-, il est traité — avant l'activation — par une amine. Dans ce cas, qui est par exemple celui de la silice ou de silicates, le support solide subit deux traitements successifs: d'abord celui de l'amination, suivi de la séparation du réactif aminé et du séchage du support; ensuite contact de ce dernier avec une solution, généralement organique, de dithioester.

En ce qui concerne l'activation préalable de l'enzyme, elle porte principalement sur la modification «douce» des résidus lysine présents à la surface externe de la protéine.

En général, le greffage du bis-dithioester sur l'enzyme a lieu dans un milieu tamponné à un pH égal ou voisin de la neutralité. Cependant, lorsque l'enzyme supporte sans inconvénient un pH légèrement basique, il y a intérêt à opérer dans un tel milieu. On accélère ainsi la réaction de greffage par catalyse due aux ions $OH^-$.

Aussi, une des formes d'exécution avantageuse de l'invention comprend-elle l'opération au sein d'un tampon de pH 7 à 9, de préférence entre pH 8 et 9.

Après la fixation d'une enzyme sur le support par lintermédiaire de bis-dithioester, il reste le plus souvent une certaine proportion de groupes dithioïques qui n'a pas réagi; il est alors indiqué de les désactiver. Pour cela, il convient, suivant l'invention, de faire réagir sur ces groupes des petites molécules du type $H_2N-R-Y$ où R est un groupe hydrocarboné aliphatique ou arylique de faible masse moléculaire, de préférence en $C_1$ à $C_8$, et Y un groupe fonctionnel choisi selon les propriétés qu'il s'agit de conférer à l'environnement de l'enzyme; Y peut être, par exemple, -COOH, $-CH_2OH$, $-CH_3$ ou autre, non nocif à l'activité de l'enzyme.

Un mode de mise en œuvre du procédé de l'invention consiste à faire d'abord l'activation des fonctions aminées portées par l'enzyme, au moyen d'un bis dithio ester, avant sa mise en contact avec le polymère aminé. Dans ce cas on optimalise au préalable la quantité de réactif nécessaire et suffisante à cette activation, pour éviter un excès de bis dithio ester. Cette façon d'opérer est avantageuse dans le

cas de certains supports aminés solubles, en évitant la réticulation qui pourrait se produire lors de l'activation directe de ces derniers.

Un exemple particulier, avantageux, du procédé d'immobilisation, suivant l'invention, consiste à traiter un gel de silice poreuse, purifiée, par une solution d'un silane dans un solvant approprié; le silane peut être un alkyl-silane, un alkyl-halogéno silane, et de préférence un alkyl-amino silane. Dans le cas où l'on n'a pas utilisé un amino-silane, le produit peut être traité par une amine ou par de l'ammoniac, après quoi le solide est séparé, lavé et séché. La silice, ainsi préactivée, est mise en suspension dans une solution organique d'un dithioester suivant l'invention; le solvant peut être par exemple un alcool, une cétone, etc. ou un mélange de solvants. Le plus souvent 0,1 à $10^{-6}$ , et surtout $10^{-2}$ à $10^{-5}$, mole de dithioester sont employées par gramme de support sec; cette quantité est déterminée à partir de la teneur en groupes $-NH_2$ du support, 1 mole de di-thioester étant employée en principe par groupe $NH_2$ présent.

Après le temps requis, compris en général entre 6 et 60 heures, le support est séparé du solvant, rincé avec celui-ci, puis à l'eau distillée, et est suspendu dans une solution tampon de pH convenant à l'enzyme que l'on envisage de fixer; cette suspension peut d'ailleur être conservé longtemps jusqu'à son utilisation.

Pour effectuer l'immobilisation, on introduit l'enzyme voulue dans la suspension du support au sein du tampon de pH; le tout est laissé au repos, en général pendant 4 à 40 heures, et le plus souvent 10 à 30 heures, à la température indiquée plus haut, de préférence vers 4° à 8°C.

Ensuite le support est séparé par filtration ou centrifugation, lavé avec une solution aqueuse d'électrolyte, puis avec un tampon de pH approprié. Il est alors prêt à l'emploi, mais peut également être conservé dans la solution tampon choisie.

Etant donné que les bis-dithioesters décrits conviennent à la fixation d'enzymes sur des supports plastiques ou pâteux, dont plusieurs exemples sont cités plus haut, ils conviennent bien à la préparation de membranes ou capteurs enzymatiques. Dans cette application la membrane peut être, par exemple, obtenue par co-réticulation d'une enzyme avec un gel de protéine, de cellulose ou d'ester de cellulose aminés, ou bien d'un polymère acrylique, styrénique ou vinylique, porteurs de groupes $NH_2$ ou NH, à l'aide d'un bisdithioester suivant la présente invention.

L'invention est illustrée par les exemples non limitatifs, qui suivent.

*Exemple 1*

*Immobilisation de glucose oxydase sur un support de silice*

100 g de silice, connue dans le commerce sous la dénomination XOB 30D, de diamètre moyen des pores de 650 Å et de surface spécifique de 50 $m^2/g$, sont d'abord traités par un mélange sulfo-nitrique 1/1, ensuite bien rincés à l'eau distillée. La silice est alors séchée jusqu'à élimination de toutes traces d'eau, puis elle est traitée pendant 6 heures à la température ambiante avec 300 ml de solution toluénique renfermant 15 g de triméthoxy propyl amino silane $(CH_3O)_3$ Si $CH_2CH_2CH_2NH_2$. La silice, ainsi silanée et aminée, est séparée de la solution, puis lavée abondamment au toluène et à l'hexane, pour éliminer le silane en excès. Elle est ensuite séchée.

5 g de cette silice aminée, sèche, sont mis en suspension dans 50 ml d'une solution éthanolique de 0,177 g, soit 0,0005 mole ($10^{-2}$ mole/litre) de tétrathiooctanedioate de carboxyméthyle

$$\mathrm{HOOC\text{-}CH_2\text{-}S\text{-}\underset{\underset{S}{\|}}{C}\text{-}(CH_2)_6\text{-}\underset{\underset{S}{\|}}{C}\text{-}S\text{-}CH_2\text{-}COOH}$$

(M = 354)

La suspension, agitée de temps en temps, est laissée à la température ambiante durant 48 heures. La silice est ensuite séparée de la solution, rincée à l'éthanol, puis à l'eau distillée, et introduite dans une solution tampon au phosphate, de pH 7, dans laquelle elle est conservée. 1 g de silice, ainsi stockée, avec 10 ml de tempon, sont additionnés de 0,020 g de glucose oxydase (GOD) du type connu sous la désignation II (sigma). On laisse le greffage s'effectuer à une température de 5° à 6°C. Après 24 heures de contact, la silice est séparée par filtration, lavée avec 20 ml d'une solution 1M de NaCl, puis avec 20 à 40 ml d'eau distillée, et enfin rincée avec la solution tampon dans laquelle a eu lieu le greffage. Le lavage à NaCl (ou urée M utilisée dans d'autres exemples) avait pour but d'éliminer la fraction d'enzyme GOD retenue seulement par adsorption.

Pour mesurer l'activité de la GOD, et ainsi confirmer sa fixation, la silice greffée est mise en contact avec une solution de glucose à 1 g/l, et son activité est déterminée par spectrophotométrie.

*Exemple 2*

*Détermination de l'activité enzymatique de la glucose oxydase immobilisée dans l'exemple 1*

On détermine tout d'abord l'activité de la même quantité d'enzyme utilisée pour le greffage, à l'état libre, en solution dans du tempon.

Cette activité de la GOD libre est déterminée à une température de 34-35°C à l'air libre, sous légère agitation, par simple contact entre l'enzyme et une solution de glucose.

L'activité de la GOD greffée sur silice est déterminée dans les mêmes conditions opératoires.

| *GOD libre* | *GOD greffée* |
|---|---|
| 20 mg GOD<br>10 ml tampon,<br>× ml de solution<br>glucose | 1 g de silice greffée<br>avec 20 mg de GOD,<br>10 ml tampon<br>× ml de solution glucose. |

L'activité dans un cas comme dans l'autre est suivie par dosages spectrophotométriques, par la méthode à l'hexokinase.

L'activité de la GOD immobilisée est pratiquement la même que celle de la GOD libre, déterminée dans les mêmes conditions.

*Exemple 3*

*Activité de l'enzyme immobilisée en fonction du temps*

Différentes déterminations d'activité enzymatique ont été effectuées sur plusieurs mois, à partir du même échantillon de glucose oxydase immobilisée sur 1,00 g de silice activée avec du tétrathiodécanedioate de carboxyméthyle greffée avec 20 ml de GOD dans 10 ml de tempon pH 7. Une solution de β-D-glucose à 5 g/l était additionnée au temps 0. Au bout d'une minute, on déterminait la quantité de glucose présente dans la suspension, de façon à déduire la proportion de substrat transformé durant ce temps. Après 6 mois de conservation l'activité était inchangée.

*Exemple 4*

*Activation préalable de l'enzyme*

A une solution de glucose oxades dans le tampon phosphate, telle que définie à l'exemple 1, on ajoute des quantités croissantes de solution de bis dithio esters ci-dessous, à la concentration de $10^{-2}$M:

(a) $HOOCH_2C$-S-C-$(CH_2)_{10}$-C-S-$CH_2$-COOH
‖ ‖
S S

(b) HOOC-$CH_2$S-C-$CH_2CH_2O$-$CH_2CH_2$-C-S$CH_2$COOH
‖ ‖
S S

Dans chaque cas on suit par spectrophotométrie à 310 nm la disparition de la fonction dithioester pour s'assurer que la réaction a eu lieu avec les groupes $NH_2$ accessibles de la glucose oxydase.

On observe une différence de réactivité des deux composés (a) et (b); ramené à la molécule de protéine active présente en solution, on peut estimer à 5 le nombre de fonctions amines activées par le composé (a) et à 3,8 par le composé (b).

En maintenant pendant 48 heures le contact entre la solution de glucose oxydase et le bis dithio ester (a) on mesure l'activité de l'enzyme après addition de son substrat et l'on n'observe aucune diminution d'activité par rapport à une solution témoin conservé dans les mêmes conditions, mais en l'absence de bis dithio ester.

Cet exemple montre que l'on peut activer en premier lieu indifféremment l'enzyme ou le support aminé, ce qui permet de choisir pour chaque type d'enzyme la démarche la mieux adaptée pour l'obtention d'un rendement optimal.

*Exemple 5*

*Immobilisation de l'alcool déshydrogénase*

10 g de silice, aminée suivant la méthode décrite à l'exemple 1 et activée par du tétra thio octane dioate de carboxyméthyle, sont maintenus pendant 48 h en suspension dans 100 ml de tampon phosphate 50 mM pH 7, contenant 100 mg d'alcool désydrogénase de levure d'activité environ 70 UI/mg.

On introduit ainsi un grand excès d'enzyme pour accroître l'activité de la préparation, l'excès d'enzyme en solution peut, après séparation du support activé, être recyclé pour une autre immobilisation.

Après 48 heures de contact à 4 °C, on sépare la silice qui est rincée successivement par du tampon phosphate, de l'eau distillée, puis du NaCl 1M, et remise en suspension dans du tampon phosphate.

On incube alors la suspension de silice greffée à l'alcool déshydrogénase, dans une solution de glycine 0,1 M pH 7,5, afin de désactiver les groupes dithioesters présents sur la silice, qui n'ont pas réagi avec l'enzyme. On lave à nouveau la préparation d'enzyme immobilisée par du tampon phosphate pH 7.

Cette préparation présente une activité spécifique 11,3 UI par gramme de silice; cette activité est remarquablement stable, comme le montrent les résultats ci-dessous, obtenus en cours de la conservation de l'enzyme immobilisée, dans du tampon phosphate de pH 7 à 4 °C.

| *Age de la préparation* (jours) | *Activité spécifique* UI/g silice |
|---|---|
| 1 jour | 11,6 |
| 5 jours | 10.9 |
| 10 jours | 11,4 |

**Revendications**

1. Procédé d'immobilisation d'enzymes sur des supports par l'intermédiaire d'un dithioester, caractérisé en ce que les deux groupes thioacide sont reliés par une groupe hydrocarboné ou éther et que la molécule de bis-dithioester porte, à chacun de ses bouts, un groupe nucléofuge capable de réagir avec une fonction amine.

2. Procédé suivant la revendication 1, caractérisé en ce que le bis-dithioester est de la forme

X-R'-S-C-R-C-S-R'X
‖ ‖
S S

où R et R' sont des groupes hydrocarbonés, aliphatiques, cycloaliphatiques ou aryliques, pouvant porter des substituants, X étant un groupe ou atome actif capable de réagir avec une fonction amine.

3. Procédé suivant la revendication 2, caractérisé

en ce que R est une chaîn3 aliphatique, de préférence en $C_2$ à $C_{20}$ , pouvant contenir des atomes d'oxygène.

4. Procédé suivant la revendication 2, caractérisé en ce que R est un aryle ou alkaryle dont le noyau peut porter un ou plusieurs substituants, en particulier halogénes, nitro, hydroxyles ou alkyles.

5. Procédé suivant la revendication 2, caractérisé en ce que R est ou renferme un cycloalkyle, en particulier cyclohexyle ou cyclopentyle, pouvant porter un ou plusieurs substituants, notamment alkyles, halogènes, nitro ou hydroxyles.

6. Procédé suivant une des revendications 2 à 5, caractérisé en ce que R' est une chaîne $-(CH_2)_n-$ avec n égal de préférence à 2 à 18, pouvant porter des substituants, en particulier alkyles, hydroxyles, nitro ou halogènes.

7. Procédé suivant une des revendications 2 à 5, caractérisé en ce que R' est un aryle, oxyaryle ou alkaryle, dont le noyau peut porter des substituants, notamment des halogènes, alkyles, nitro, sulfo, hydroxy, oxy ou thiol.

8. Procédé suivant une des revendications 2 à 7, caractérisé en ce que X est un carboxyle.

9. Procédé suivant une des revendications 2 à 7, caractérisé en ce que X est constitué par un ou plusieurs halogènes, hydroxyles, sulfonyles, sulfinyles, phosphoryles ou autres groupes oxygénés du phosphore, dialyklamino ou mercapto.

10. Application du procédé suivant une des revendications précédentes à la fixation d'hydrolases, d'oxydoréductases, de transférases, de lyases, d'isomérases ou/et de ligases sur des supports solides, plastiques ou pâteaux, inorganiques ou organiques, porteurs de groupes amino, en vue de l'utilisation de l'enzyme, ainsi immobilisée, dans un réacteur ou dans une membrane enzymatique.

11. Procédé suivant une des revendications précédentes, caractérisé en ce que l'activation préalable, au moyen d'un bis-dithioester, est appliquée à l'enzyme.

**Patentansprüche**

1. Verfahren zur Immobilisierung von Enzymen auf Trägern mittels eines Dithioesters, dadurch gekennzeichnet, dass die beiden Thiosäuregruppen durch eine Kohlenwasserstoff- oder Ethergruppe verbunden sind und das Molekül des Bis-dithioesters an jedem seiner Enden eine nucleofuge Gruppe trägt, die in der Lage ist, mit einer Aminofunktion zu reagieren.

2. Verfahren nach Anspruch 1, dadurch gekennzeichnet, dass der Bis-dithioester die folgende Formel aufweist

$$\begin{array}{c} X\text{-}R'\text{-}S\text{-}\underset{\displaystyle S}{\underset{\|}{C}}\text{-}R\text{-}\underset{\displaystyle S}{\underset{\|}{C}}\text{-}S\text{-}R'X \end{array}$$

worin R und R' aliphatische, cycloaliphatische oder Arylkohlenwasserstoffgruppen bedeuten, die Substituenten aufweisen können und X eine aktive Gruppe oder Atom ist, die mit einer Aminfunktion reagieren können.

3. Verfahren nach Anspruch 2, dadurch gekennzeichnet, dass R eine aliphatische Kette, vorzugsweise mit 2 bis 20 C-Atomen ist, die Sauerstoffatome enthalten kann.

4. Verfahren nach Anspruch 2, dadurch gekennzeichnet, dass R ein Aryl- oder Alkarylrest ist, dessen Kern einen oder mehrere Substituenten, insbesondere Halogenatome, Nitro-, Hydroxyl- oder Alkylreste, aufweisen kann.

5. Verfahren nach Anspruch 2, dadurch gekennzeichnet, dass R einen Cycloalkylrest, insbesondere einen Cyclohexyl- oder Cyclopentylrest, darstellt oder diesen einschliesst, der einen oder mehrere Substituenten, insbesondere Alkyreste, Halogenatome, Nitro- oder Hydroxylreste, aufweisen kann.

6. Verfahren nach einem der Ansprüche 2 bis 5, dadurch gekennzeichnet, dass R' eine Kette $-(CH_2)_n-$ ist, in der n vorzugsweise einen Wert von 2 bis 18 hat und die Substituenten, insbesondere Alkyl-, Hydroxyl-, Nitroreste oder Halogenatome, aufweisen kann.

7. Verfahren nach einem der Ansprüche 2 bis 5, dadurch gekennzeichnet, dass R' einen Aryl-, Oxyaryl- oder Alkarylrest bedeutet, deren Kern Substituenten, insbesondere Halogenatome, Alkyl-, Nitro-, Sulfo-, Hydroxy-, Oxy- oder Thiolreste aufweisen kann.

8. Verfahren nach einem der Ansprüche 2 bis 7, dadurch gekennzeichnet, dass X einen Carboxylrest bedeutet.

9. Verfahren nach einem der Ansprüche 2 bis 7, dadurch gekennzeichnet, dass X einen oder mehrere Halogenatome, Hydroxyl-, Sulfonyl-, Sulfinyl, Phosphoryl- oder andere oxygenierte Phosphorreste, Dialkylamino- oder Mercaptoreste bedeutet.

10. Anwendung des Verfahrens nach einem der vorstehenden Ansprüche zur Fixierung von Hydrolasen, Oxidoreduktasen, Transferasen, Lyasen, Isomerasen oder/und Ligasen auf festen, plastischen oder pastenartigen anorganischen oder organischen Trägern, die Aminogruppen aufweisen, um das auf diese Weise immobilisierte Enzym in einem Enzymreaktor oder -membran zu verwenden.

11. Verfahren nach einem der vorstehenden Ansprüche, dadurch gekennzeichnet, dass die vorherige Aktivierung mittels eines Bis-dithioesters auf das Enzym angewandt wird.

**Claims**

1. Process of immobilisation of enzymes on substrates by means of a dithioester, characterised in that the two thioacid groups are connected by a hydrocarbon or ether group and that the bis-dithioester molecule carries, at each of its ends, a nucleofuge group capable of reacting with an amine function.

2. Process according to claim 1, characterised in that the bis-dithioester is of the form

$$\begin{array}{c} X\text{-}R'\text{-}S\text{-}\underset{\displaystyle S}{\underset{\|}{C}}\text{-}R\text{-}\underset{\displaystyle S}{\underset{\|}{C}}\text{-}S\text{-}R'X \end{array}$$

where R and R' are aliphatic, cycloaliphatic or aryl

hydrocarbon groups, which can carry substituents, X being an active groups or atom capable of reacting with an amine function.

3. Process according to claim 2, characterised in that R is an aliphatic chain, preferably $C_2$ to $C_{20}$, which can contain oxygen atoms.

4. Process according to claim 2, characterised in that R is an aryl or alkaryl the ring of which can carry one or more substituents, in particular halogens, nitro hydroxyls or alkyls.

5. Process according to claim 2, characterised in that R is or includes a cycloalkyl, in particular cyclohexyl or cyclopentyl, which can carry one or more substituents, particularly alkyls, halogens, nitro or hydroxyls.

6. Process according to any of claims 2 to 5, characterised in that R' is a $-(CH_2)_n-$ chain with n preferably equal to 2 to 18, which can carry substituents, in particular alkyls, hydroxyls, nitro or halogens.

7. Process according to any of claims 2 to 5, characterised in that R' is an aryl, oxyaryl or alkaryl, the ring of which can carry substituents, particularly halogens, alkyls, nitro, sulpho, hydroxy, oxy or thiol.

8. Process according to any of claims 2 to 7, characterised in that X is a carboxyl.

9. Process according to any of claims 2 to 7, characterised in that X comprises one or more halogens, hydroxyls, sulphonyls, sulphinyls, phosphoryls or other oxygen-containing phosphorus groups, dialkylamino or mercapto.

10. Use of the process according to any of the preceding claims for the fixation of hydrolases, oxydoreductases, transferases, lyases, isomerases and/or ligases upon solid, plastic or pasty inorganic or organic substrates, carrying amino groups, for use of the thus immobilised enzymes in a reactor or in an enzymatic membrane.

11. Process according to any of the preceding claims, characterised in that preliminary activation by means of a bis-dithioester is carried out on the enzyme.